# EUROPÄISCHE PATENTSCHRIFT

(11) **EP 2 663 253 B1**
(45) Veröffentlichungstag und Bekanntmachung des Hinweises auf die Patenterteilung: **31.12.2014**
(21) Anmeldenummer: 12706438.4
(22) Anmeldetag: 11.01.2012
(51) Int. Cl.: A61B 19/00

(54) **OPERATIONSHALTERUNG MIT SCHNELL WECHSELBARER HALTEPLATTE FÜR DIE DRUCKELEMENTE**
SURGICAL FIXTURE DEVICE HAVING A RAPIDLY EXCHANGEABLE RETAINING PLATE FOR THE PRESSURE ELEMENTS
DISPOSITIF DE MAINTIEN POUR OPÉRATIONS COMPORTANT UNE PLAQUE DE MAINTIEN REMPLAÇABLE POUR LES ÉLÉMENTS DE PRESSION

(30) Priorität: 13.01.2011 DE 102011008490
(43) Veröffentlichungstag der Anmeldung: 20.11.2013
(73) Patentinhaber: Noras, Hubert, 97082 Würzburg (DE)
(72) Erfinder: Noras, Hubert, 97082 Würzburg (DE)
(74) Vertreter: Pöhner, Wilfried Anton
(86) Internationale Anmeldenummer: PCT/DE2012/000016
(87) Internationale Veröffentlichungsnummer: WO 2012/095102

(56) Entgegenhaltungen:
- US-A1- 2004 123 870

## Beschreibung

Die Erfindung bezieht sich auf eine Operationshalterung, bestehend aus einem etwa C-förmigen oder U-förmigen Haltebügel, der auf einem Teil des äußeren Umfanges von einem Körperteil verläuft und der zum Körperteil beabstandet ist und der am Ende seines ersten Schenkels wenigstens ein einziges Druckelement trägt und der am Ende seines zweiten Schenkels wenigstens zwei Druckelemente trägt und alle Druckelemente auf die Oberfläche des Körperteils aufgedrückt sind.

Bei Operationen oder anderen Eingriffen an Körperteilen wie dem Kopf oder dem Arm oder einem Bein oder bei Kindern sogar dem Rumpf ist es erforderlich, das zu behandelnde Körperteil fest einzuspannen. Diese Position muss über die gesamte Behandlung hinweg mit großer Genauigkeit erhalten bleiben.

Ein sehr interessantes Anwendungsbeispiel sind Schädeloperationen zur Entfernung von Gehirntumoren. Da der Operateur das befallene Gewebe mit bloßem Auge nicht von gesundem Gewebe unterscheiden kann, muss der Patient während des Eingriffes in eine Diagnoseeinrichtung wie z. B. ein CT oder MRT verfahren werden. Durch diese bildgebenden Diagnosegeräte wird der bereits operierte Bereich nochmals daraufhin überprüft, ob die Entfernung von weiterem krankem Material erforderlich ist. Bei diesem Diagnoseschritt werden die Koordinaten des noch zu entfernenden Bereiches über einige Eckpunkte erfasst.

Nach dem Herausfahren des Körperteiles aus der Diagnoseeinrichtung sind die ermittelten Werte für die Koordinaten das einzige Hilfsmittel, um die zu entfernenden Bereiche korrekt zu identifizieren. Deshalb ist es von ganz eminenter Bedeutung, dass der zu behandelnde Körperteil während des Verfahrens in seiner Position gegenüber der Operationshalterung verbleibt.

Auf aktuellem Stand der Technik sind Haltebügel bekannt, die an ihren Enden Bohrungen oder Gewindebohrungen aufweisen, in denen die Druckelemente verschraubt sind. So beschreibt z. B. das Patent US 5,254,079, Agbodoe, einen Haltebügel für Schädeloperationen. Am Ende des einen Schenkels ist eine Öse angeformt, in welche ein Gewinde eingeschnitten ist, in das ein Innengewinde einschnitten ist, in welches ein länglicher Schädeldorn mit einem komplementären Außengewinde eingeschraubt ist. Dieses Gewinde erstreckt sich über fast die gesamte Länge des Schädeldorns. Daraus ergibt sich der Nachteil, dass ein Wechsel des Schädeldorns eine relativ lange Zeit zum Herausdrehen des Außengewindes aus dem Innengewinde erfordern kann.

Am Ende des anderen Schenkels von Haltebügel ist eine kleine Bohrung eingebracht, in welche ein weiterer, kleiner Hilfshaltebügel eingeschraubt wird. Dieser kleine Hilfshaltebügel trägt an seinen beiden Enden je einen Schädeldorn, die beide in einem bestimmten Winkel zueinander geneigt sind. Wenn es erforderlich ist, dass der Neigungswinkel der beiden Schädeldorne gegeneinander geändert werden muss - z. B. weil anstatt des Schädels eines Erwachsenen der Kopf eines Kindes eingespannt werden muss - so muss der Hilfshaltebügel ausgewechselt werden.

Nachteiligerweise muss dazu die Schraubverbindung komplett gelöst werden, die Schraube entnommen werden und dann der Hilfshaltebügel gewechselt werden. Danach muss die Schraube zentriert werden, sodass sie ohne Beschädigung des Gewindes eingedreht werden kann. Anschließend ist für das Eindrehen und das Festziehen der Schraube eine weitere Bearbeitungszeit erforderlich. Insgesamt ist also eine relativ sehr lange Zeit für das Wechseln eines Schädeldornes erforderlich.

Aus der US 2004/0123870 ist eine Operationshalterung gemäß der Präambel von Anspruch 1 bekannt, bei welcher Schrauben als Druckelemente eingesetzt werden, die auf die Oberfläche des zu untersuchenden Körperteiles einwirken und die einer Kopfplatte befestigt sind. Eine Anpassung an unterschiedliche Oberflachengeometrien des zu untersuchenden Körperteils erfordert ein langwieriges Verstellen der Druckelemente oder gar die vollständige Demontage und den Austausch der die Druckelemente tragenden Kopfplatte.

Da in der Praxis häufig erst nach dem Auflegen des Patienten und dessen erster Justierung abschätzbar ist, welcher Typ von Schädeldorn am besten geeignet ist, kann ein schneller Wechsel des Schädeldorns erforderlich werden. Die dafür aufzuwendende Zeit ist bei Operationen in der Regel sehr kostenintensiv, da während des Schraubens an den Schädeldornen das übrige Operationsteam warten muss und weil der Patient solange anästhesiert werden muss und der Operationssaal für andere Verwendungen blockiert ist.

Auf diesem Hintergrund hat sich die Erfindung die Aufgabe gestellt, eine Verbindung zwischen Druckelementen - wie z. B. Schädeldornen - und dem sie tragenden Haltebügel zu entwickeln, die einen sehr schnellen Wechsel der Druckelemente erlauben und das auch bei bereits aufliegendem Patient.

Als Lösung lehrt die die in Anspruch 1 definierte Erfindung, dass in das Hoblprofil eine Halteplatte eingebracht ist, in die die wenigstens zwei Gewindebohrungen eingebracht sind.

Ein wesentliches Merkmal der Erfindung ist also, dass für die Verbindung zwischen dem Haltebügel und den Druckelementen nicht ein einziges Verbindungsstück eingesetzt wird, sondern am Haltebügel eine universell verwendbare Kopfplatte befestigt ist. Mit der Kopfplatte werden die Schädeldorne verbunden, indem sie auf einer Halteplatte befestigt sind, welche - in sehr kurzer Zeit und mit nur einem einzigen Handgriff - in die Kopfplatte einschiebbar oder einsteckbar ist. Es ist also das Verdienst der Erfindung, durch die Verlagerung der Verbindungsaufgabe auf zwei, sich berührende Platten ausreichenden Freiraum für eine schnell lösbare Verbindung geschaffen zu haben.

Dabei dient das Hohlprofil im allgemeinsten Fall nur zur Führung und Zentrierung der Halteplatte auf dem letzten Teilstück der Annäherung an die Kopfplatte. In einer vorteilhaften Ausführungsform kann das Hohlprofil jedoch auch zu einer vollständigen Verbindung ausgebaut werden.

Die Erfindung schlägt als eine vorteilhafte Ausführungsform ein etwa U-förmiges Hohlprofil vor, in dessen Seitenwände je eine Vertiefung eingebracht ist, die in Längsrichtung der Druckelemente eine Hinterschneidung bilden. Die dazu passende Halteplatte weist im Querschnitt ein zum Hohlprofil zumindest teilweise komplementäres Profil auf, das mit seitlichen Auswölbungen in die Vertiefungen der Seitenwände des Hohlprofils eingreift. Diese Auswölbungen laufen im Normalfall über die beiden seitenkanten der Halteplatte hinweg durch. Es ist jedoch auch möglich, diese Auswölbungen nur Abschnittsweise an die Seitenkanten der Halteplatte anzuformen.

Halteplatten mit beiden Varianten sind in die Stirnseiten des Hohlprofils einschiebbar, wobei die Auswölbungen an den Kanten sich in die Vertiefungen der Seitenwände des Hohlprofils einschmiegen. Dadurch wird die Halteplatte beim Einschieben in die Kopfplatte geführt und ist beim Erreichen ihrer Endposition gegen ein Herausfallen abgesichert, weil die Vertiefungen in Längsrichtung der Druckelemente gesehen eine Hinterschneidung bilden.

Um das Einführen der Halteplatte in das Hohlprofil zu erleichtern, schlägt die Erfindung vor, dass das Profil der Halteplatte an beiden Enden angeschrägt ist, sodass es zu Beginn des Einführungsvorganges noch ein sehr großes Spiel zu dem Hohlprofil hat. Dadurch wird der einführenden Person zu Beginn des Vorganges nur eine relativ niedrige Positioniergenauigkeit abverlangt, was den Vorgang erheblich beschleunigt.

Die "angeschrägten" Abschnitte der Halteplatte können zwar nicht mehr zum Rückhalt der Halteplatte in der Kopfplatte beitragen, aber diese Reduzierung bleibt deshalb ohne Folgen, da die erforderlichen Rückhaltekräfte nur durch das Eigengewicht der Halteplatte und der darauf befestigten Schädeldorne bestimmt werden. Bei der Aktivierung der Schädeldorne hingegen wird die Halteplatte mit ihrer gesamten rückseitigen Fläche in die Grundfläche des Hohlprofils gedrückt, wo sie eine vergleichsweise sehr große Auflagefläche vorfindet, sodass eine Ableitung dieser Kräfte gesichert ist.

Da es wünschenswert ist, dass der Halteplatte beim Einschieben in die Kopfplatte nur sehr geringe Kräfte entgegengesetzt werden, damit die Zeitdauer des Einschiebens so gering wie nur möglich ist, könnte diese "leichte" Verschiebbarkeit womöglich ein nicht beabsichtigtes Verrutschen der Halteplatte gegenüber der Kopfplatte verursachen. Um das zu verhindern, schlägt die Erfindung in einer weiteren Ausführungsvariante vor, dass an den beiden Stirnseiten der Halteplatte je eine federnde Rastnase angeformt oder befestigt wird, die nach dem vollständigen Einschieben der Halteplatte in die Kopfplatte auf jeweils einer der beiden Stirnflächen der Kopfplatte aufliegt. Zum Herausschieben der Halteplatte aus der Kopfplatte muss eine dieser beiden Rastnasen von der Kopfplatte abgehoben werden, sodass sie durch das Hohlprofil hindurch aus der Kopfplatte herausgleitet.

Die Erfindung bevorzugt ein durch die Kopfplatte vollständig hindurchlaufendes Profil, sodass die Halteplatte an beiden Stirnseiten der Kopfplatte eingesteckt und auch wieder herausgezogen werden kann.

Prinzipiell ist jedoch auch eine Variante denkbar, bei der ein Ende des Hohlprofils mit einem Anschlag für die Halteplatte verschlossen ist. Dann würde nur eine einzige federnde Rastnase benötigt, die in eine dazu komplementäre Vertiefung bzw. auf eine aus der Kopfplatte herausragende Auswölbung einrasten muss. Beim Herausnehmen der Halteplatte aus der Kopfplatte ist diese Rastverbindung durch Anheben des Rastelementes wieder zu deaktivieren.

In der Praxis ist es wünschenswert, dass die Oberfläche des zu fixierenden Körperteils in der Nähe des Schädeldorns verschiedene Neigungen in Bezug auf den Endpunkt des Haltebügels haben kann. Daraus folgt der Wunsch, dass die Kopfplatte gegenüber dem Haltebügel in zwei Richtungen verschwenkbar sein sollte. Um diese Freiheit bei der Verstellung zu erreichen, schlägt die Erfindung vor, dass die Kopfplatte eine kugelsegmentförmige Ausnehmung aufweist, welche auf einen Kugelkopf aufgesetzt werden kann und darauf in einer wählbaren Winkelposition fixiert werden kann.

Zur Fixierung ist z. B. eine Schraube geeignet, die durch die kugelsegmentförmige Ausnehmung sowie durch den Kugelkopf hindurch verläuft. Dafür ist in einem der beiden Elemente "Kugelkopf" oder "kugelsegmentförmige Ausnehmung" eine Öffnung erforderlich, die erheblich größer als der Querschnitt der Schraube ist. In dem anderen, dazu komplementären Bauteil muss die Öffnung nur gerade so groß sein, dass die Schraube glatt hindurch gesteckt werden kann.

Anstelle der Schraube kann auch ein Bolzen verwendet werden, der an seinem einen Ende einen radial überkragenden Kopf aufweist und der am anderen Ende mit einer Hebelkonstruktion verbunden ist, die sich an einem Teil des Haltebügels abstützt. Dann ist es möglich, die Verbindung zwischen Kugelkopf und Kugelsegment schnell zu lockern, die Kopfplatte in den gewünschten Winkel zu schwenken und zwar sowohl in horizontaler wie auch in vertikaler Richtung und anschließend wieder mit einem Handgriff festzuklemmen.

Falls auf die Verbindung zwischen Kopfplatte und Haltebügel besonders hohe Kräfte einwirken oder besonders hohe Kraftimpulse möglich sein müssen, schlägt die Erfindung vor, dass auf den Kugelkopf Rippen oder Wülste oder Noppen aufgeformt sind, die in dazu komplementäre Vertiefungen in der kugelsegmentförmigen Ausnehmung der Halteplatte eingreifen. Wenn diese Elemente jeweils in einem gleichbleibenden Raster angeordnet sind, dann ist damit eine stufenweise Verschwenkung der Kopfplatte gegenüber dem Haltebügel möglich.

Bei gleichmäßig durchlaufenden Rippen ist eine Verschwenkung in tangentialer Richtung zu den Rippen auch weiterhin stufenlos möglich.

In einer anderen Ausführungsvariante weist die Halteplatte kein Profil auf, das zum Hohlprofil der Kopfplatte komplementär ist, sondern wenigstens einen Stift, der in eine dazu komplementäre Bohrung in der Kopfplatte einsteckbar und/oder einrastbar ist. In diesem Fall benötigt das Hohlprofil keine Vertiefungen in seinen Seitenwänden sondern benutzt seine Seitenwände lediglich dazu, die Halteplatte beim Einführen in die Kopfplatte zu zentrieren und gegen seitliches Abgleiten zu sichern.

Es ist ein weiteres Merkmal der Kopfplatte, dass sie alternativ auch ohne Halteplatte zur Befestigung von Druckelementen dienen kann. Dazu weist sie zwei Gewindebohrungen mit je einem Innengewinde auf, in welche je ein Druckelement eingeschraubt werden kann. Wenn die Länge dieses Gewindeganges relativ kurz ist, so ist die zum Einsetzen und Wechseln erforderliche Zeit trotzdem relativ kurz. Besonders vorteilhaft ist es, wenn diese Gewindebohrung von der Rückseite her zugänglich ist, sodass das Druckelement von der Rückseite der Kopfplatte an das Körperteil herangeführt werden kann.

Diese Variante ist insbesondere für die relativ sehr schmalen Schädeldorne sinnvoll, da diese als Druckelement eine kegelförmige Spitze aufweisen, die sich in die Oberfläche des Körperteiles - also z. B. in die Schädeldecke des Kopfes - hineindrückt. Der Vorteil eines solchen Dornes ist die sehr gute Fixierung, die über die Dauer der gesamten Operation hinweg stabil bleibt sowie die vergleichsweise geringe, nur punktförmige Verletzung der Haut. Diese kann mitunter sogar geringer sein als bei einem großflächig aufliegenden Kissen, das bei längeren Operationen für das Abquetschen der Blutgefäße der Haut sorgt und dadurch zu einem Absterben der beaufschlagten Hautpartie beitragen kann.

Im Folgenden sollen weitere Einzelheiten und Merkmale der Erfindung anhand eines Beispiels näher erläutert werden. Dieses soll die Erfindung jedoch nicht einschränken, sondern nur erläutern. Es zeigt in schematischer Darstellung:
- Figur 1: Schrägbild einer Operationshalterung für einen Schädel
- Figur 2: Schrägbild einer Kopfplatte mit eingeschobener Halteplatte
- Figur 3: Kopfplatte wie Figur 2, jedoch ohne Halteplatte
- Figur 4: Kopfplatte ähnlich zu Figur 3 mit eingeschraubten Druckelementen
- Figur 5: Halteplatte mit Stiften und dazu komplementärer Kopfplatte 4

In der folgenden Beschreibung werden sowohl für das Körperteil 1 als auch für den Schädel 1, als eines von vielen anderen Beispielen für ein Körperteil 1 jeweils das gleiche Bezugszeichen 1 verwendet. Ebenso wird auch der Schädeldorn 3 mit dem Bezugszeichen 3 versehen, obwohl er nur eine von vielen Ausführungsformen eines Druckelementes 3 ist.

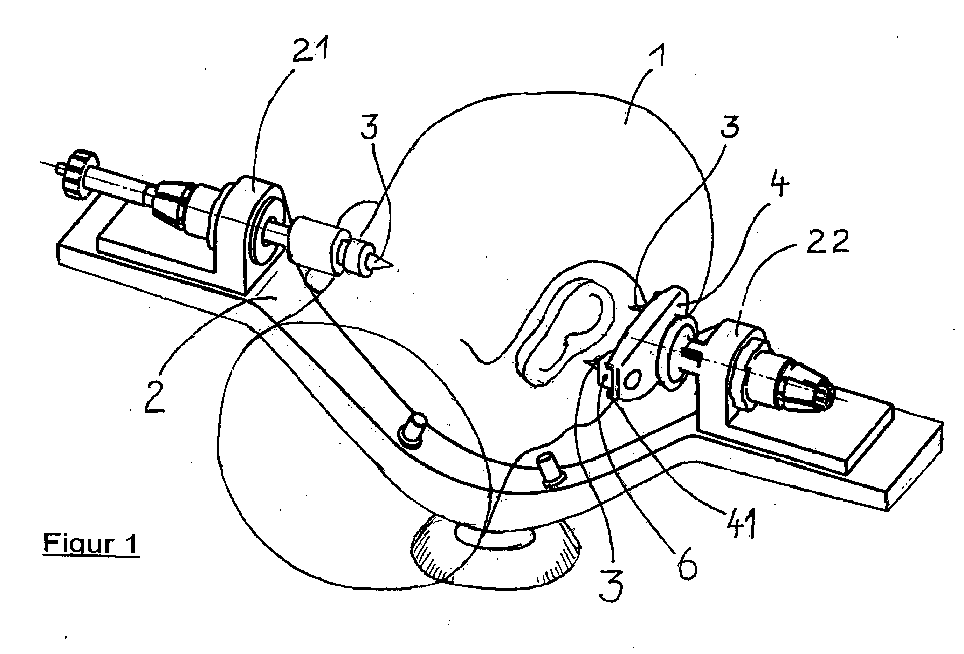

In Figur 1 ist als Körperteil 1 ein Schädel 1 gezeichnet, der für eine Schädeloperation in einen Haltebügel 2 eingespannt ist.

Zur besseren Erkennbarkeit des Haltebügels 2 und des linksseitig in den Schädel 1 eindringenden Druckelementes 3, ist in Figur 1 der Schädel 1 gläsern dargestellt. Dadurch wird deutlich erkennbar, dass er an seiner linken Seite von einem einzigen Druckelement 3 - hier einem Schädeldorn 3 - gehalten wird und an seiner rechten Seite von zwei Schädeldornen 3. Während der links dargestellte Schädeldorn 3 in konventioneller und bekannter Methode direkt in einer Halterung auf dem ersten Schenkel 21 des Haltebügels 2 gehalten wird - hier sogar mit einer Kraftmesseinrichtung zwischen Schädeldorn 3 und dem ersten Schenkel 21 - werden auf der rechten Seite die beiden Schädeldorne 3 von einer Halteplatte 6 gehalten. Diese Halteplatte 6 ist seitlich in das Hohlprofil 41 der Kopfplatte 4 eingeschoben. Die Kopfplatte 4 ist über einen Bolzen am zweiten Schenkel 22 des Haltebügels 2 befestigt.

In Figur 1 wird sofort deutlich, dass nur eine geringfügige Lockerung des Druckes von der Kopfplatte 4 auf den Schädel 1 erforderlich ist, um die Halteplatte 6 aus dem Hohlprofil 41 in der Kopfplatte 4 herauszuziehen. Die dafür erforderliche Bewegung der Halteplatte 6 mit den darauf befestigten Schädeldornen 3 erfolgt in Längsrichtung des Schädels 1, wofür ausreichend Platz vorhanden ist, wie in Figur 1 sofort ersichtlich ist.

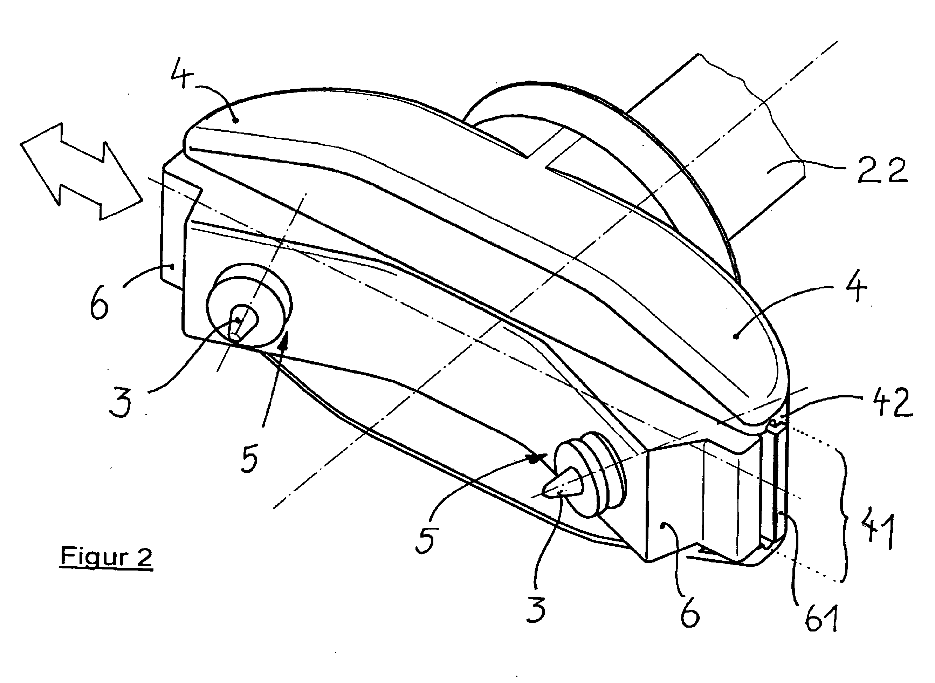

In Figur 2 ist eine Kopfplatte 4 von der in Figur 1 nicht sichtbaren, zum Schädel 1 weisenden Seite dargestellt. Sehr gut zu erkennen ist, dass in die Kopfplatte 4 eine Halteplatte 6 eingeschoben ist, die mit zwei Schädeldornen 3 bestückt ist. In Figur 1 ist mit jeweils einem dünnen Pfeil auf die beiden Gewindebohrungen 5 hingewiesen, die in die Halteplatte 6 eingebracht sind, um darin die Schädeldorne 3 einzuschrauben.

An der rechten Seite der Kopfplatte 4 ist das Hohlprofil 41 mit seinen beiden - hier etwa halbkreisförmigen - Vertiefungen in seinen beiden gegenüberliegenden Seitenwänden zu erkennen. In Figur 2 wird dadurch sehr gut nachvollziehbar, wie die Halteplatte 6 mit zwei Wülsten an ihren Seiten in diese Vertiefungen hineinfasst und darin quer zur Längsachse der beiden Schädeldorne 3 verschoben werden kann. Diese Verschiebungsbewegung ist an der linken Seite von Figur 2 durch einen großen Doppelpfeil wiedergegeben.

An der rechten Seite von Figur 2 ist an der Stirnseite der Halteplatte 6 die Rastnase 61 zu erkennen. In Figur 2 ist sehr schön sichtbar, wie diese Rastnase 61 mit ihrem abgewinkeltem Endbereich auf der Stirnfläche 42 der Kopfplatte 4 aufliegt. Dadurch blockiert dieser Endbereich eine Bewegung der Halteplatte 6 nach links. Da an der anderen Stirnseite der Halteplatte ebenfalls eine - in Figur 2 nicht sichtbare - Rastnase 61 angeordnet ist, wird auch eine - unbeab-sichtigte - Verschiebung der Halteplatte 6 nach rechts blockiert.

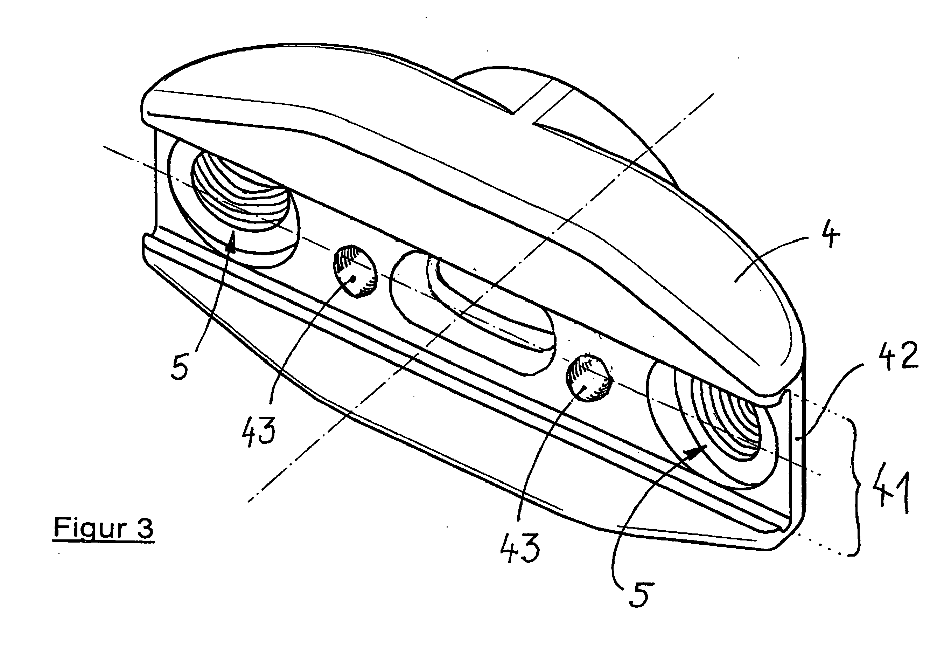

In Figur 3 ist eine Kopfplatte 4 als Einzelteil dargestellt. Darin ist sehr schön zu erkennen, wie das Hohlprofil 41 durch die gesamte Kopfplatte 4 gleichmäßig hinwegläuft. Deutlich wird auch, dass das Hohlprofil 41 in dieser Ausführungsform U-förmig ist und an den beiden gegenüberliegenden Seitenwänden des U je eine rinnenförmige Vertiefung eingeprägt ist. Auch in Figur 3 ist gut nachvollziehbar, dass in diese rinnenförmige Vertiefung ein entsprechendes, wulstförmiges Gegenstück an den beiden Kanten einer - hier nicht eingezeichneten - Halteplatte 6 einschiebbar ist.

Auf der Grundfläche des U-förmigen Hohlprofils 41 ist in der Mitte eine Öffnung angeordnet, durch welche ein Befestigungsbolzen zur Verbindung der Kopfplatte 4 mit einem Schenkel 21, 22 des Haltebügels 4 geschoben wird. Daran anschließend sind auf beiden Seiten je eine Bohrung 43 angeordnet. In einer anderen Ausführungsform einer Halteplatte 6 dienen sie zur Aufnahme von Stiften 62 die mit der Halteplatte 6 verbunden sind. Ganz am äußeren Rand des Hohlprofils 41 sind zwei relativ große Gewindebohrungen 5 zu erkennen. In einer weiteren Ausführungsvariante können in diese Gewindebohrungen 5 jeweils ein Schädeldorn 3 eingeschraubt werden, der dann ohne Zwischenschaltung einer Halteplatte 6 direkt mit der Kopfplatte 4 verbunden ist.

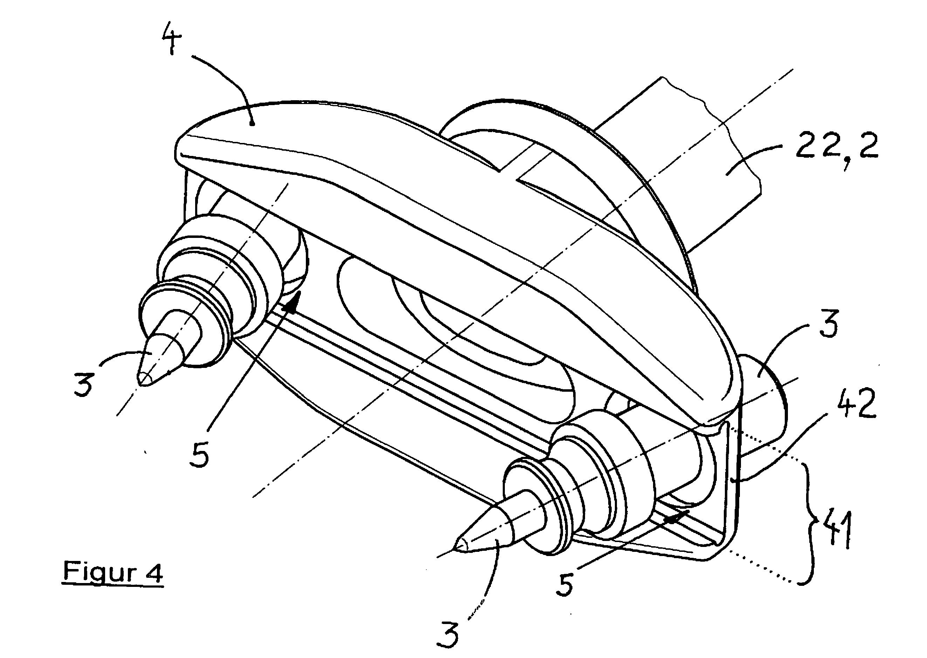

In Figur 4 ist eine geringfügig andere Variante einer Kopfplatte 4 dargestellt. Sie weist in der Mitte lediglich ein relativ großes Langloch zur Aufnahme des Bolzens für die Verbindung mit dem Haltebügel 2 auf. In dieser Variante fehlen die beiden Bohrungen 43. Stattdessen ist die mittlere Öffnung etwas größer. In Figur 4 wird nachvollziehbar, dass alternativ an die Enden des hier dargestellten Langloches ebenfalls jeweils zwei - hier nicht dargestellte - Stifte 62 einer Halteplatte 6 eingesteckt werden können, die dann nur mit einem Teil Ihres Umfanges das Langloch berühren.

In Figur 4 ist sehr deutlich zu erkennen, dass in die beiden Gewindebohrungen 5 an den beiden Enden der Kopfplatte 4 je ein relativ großer Schädeldorn 3 eingeschraubt ist. Beide Gewindebohrungen 5 sind leicht gegeneinander geneigt, so dass auch die beiden darin eingeschraubten Schädeldorne 3 zueinander geneigt verlaufen. In Figur 4 ist das Hohlprofil 41 an der rechten Seite durch eine geschweifte Klammer markiert, das jedoch in dieser Nutzungsvariante nicht zum Einsatz kommt.

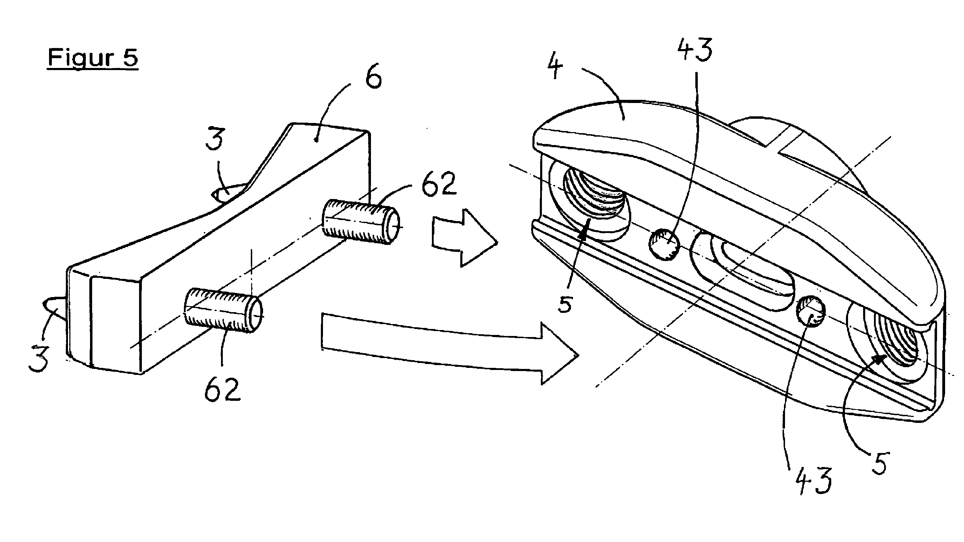

In Figur 5 ist eine weitere Variante zur Nutzung einer Kopfplatte 4 gezeigt, wobei die hier wiedergegebene Variante der Kopfplatte 4 exakt der Figur 3 entspricht.

In Figur 5 ist eine weitere Version einer zur gezeigten Kopfplatte 4 kompatiblen Halteplatte 6 zu sehen. Auf deren links dargestellter Vorderseite sind zwei Schädeldorne 3 befestigt. An der zum Betrachter weisenden Rückseite sind zwei Stifte 62 angeformt.

In Figur 5 ist mit einem kurzen und einem langen Doppelpfeil angedeutet, wie zur Befestigung der dargestellten Ausführungsform der Halteplatte 6 in der Kopfplatte 4 die beiden Stifte 62 in je eine Bohrung 43 in der Kopfplatte 4 auf dem Grund des Hohlprofiles 41 eingeführt werden und sich dort verkeilen. In Figur 5 wird deutlich, dass in dieser Nutzungsvariante die beiden Gewindebohrungen 5 unbenutzt bleiben.

### Bezugszeichenliste

- 1: Körperteil, z.B. Schädel
- 2: Haltebügel, umgibt Körperteil 1, trägt Kopfplatten 4
- 21: erster Schenkel des Haltebügels 2
- 22: zweiter Schenkel des Haltebügels 2
- 3: Druckelement, z.B. Schädeldorn, drückt auf Körperteil 1
- 4: Kopfplatte, an Haltebügel 2 befestigt
- 41: Hohlprofil in Kopfplatte 4
- 42: Stirnflächen der Kopfplatte 4
- 43: Bohrungen in Kopfplatte 4 für Stifte 62
- 5: Gewindebohrung, in Kopfplatte 4 oder in Halteplatte 6
- 6: Halteplatte, an Kopfplatte 4 montiert
- 61: Rastnasen an den Stirnseiten der Halteplatte 6
- 62: Stifte an Halteplatte 6, in Bohrungen 43 einsteckbar

## Patentansprüche

1. Operationshalterung, bestehend aus einem etwa C-förmigen oder U-förmigen Haltebügel (2),
- der geeignet ist auf einem Teil des äußeren Umfanges von einem Körperteil (1) zu verlaufen und
- zum Körperteil (1) einen Abstand aufzuweisen und
- der am Ende seines ersten Schenkels (21) wenigstens ein einziges Druckelement (3) trägt und
- der am Ende seines zweiten Schenkels (22) wenigstens zwei Druckelemente (3) trägt und
- alle Druckelemente (3) geeignet sind auf die Oberfläche des Körperteils (1) aufgedrückt oder teilweise in die Oberfläche hineingedrückt zu werden,
- die Druckelemente (3) an wenigstens einem der beiden Schenkel (21), (22) mittels je einer Kopfplatte (4) befestigt sind,
- sowie wenigstens zwei Gewindebohrungen (5) mit je einem Innengewinde vorhanden sind, in dem ein Druckelement (3) befestigt ist,
- in die Kopfplatte (4) ein etwa quer zu den Druckelementen (3) ausgerichtetes Hohlprofil (41) eingeformt ist, das derart gestaltet ist, dass verschiedene Typen von Halteplatten (6) einschiebbar oder einsteckbar sind, **dadurch gekennzeichnet, dass** in das Hohlprofil eine Halteplatte (6) eingebracht ist, in die die wenigstens zwei Gewindebohrungen (5) eingebracht sind.

2. Operationshalterung nach Anspruch 1, **dadurch gekennzeichnet, dass** das Körperteil
- der Kopf oder
- ein Arm oder
- ein Bein oder
- das Becken
ist.

3. Operationshalterung nach einem der vorhergehenden Ansprüche, **dadurch gekennzeichnet, dass** das Druckelement (3)
- ein Schädeldorn oder
- eine Andruckplatte oder
- ein Andruckkissen, wie z. B. ein Gelkissen
ist.

4. Operationshalterung nach einem der vorhergehenden Ansprüche, **dadurch gekennzeichnet, dass** das Hohlprofil (41) etwa U-förmig ist und seine Seitenwände je eine Vertiefung aufweisen, die in Längsrichtung der Druckelemente (3) eine Hinterschneidung bilden.

5. Operationshalterung nach Anspruch 4, **dadurch gekennzeichnet, dass** die Halteplatte (6) im Querschnitt ein zum Hohlprofil (41) zumindest teilweise komplementäres Profil aufweist, mit zumindest teilweise durchlaufenden Auswölbungen, die in die Vertiefungen der Seitenwände des Hohlprofils (41) eingreifen.

6. Operationshalterung nach einem Anspruch 5, **dadurch gekennzeichnet, dass** die Halteplatte (6) an ihren beiden Stirnseiten federnde Rastnasen (61) aufweist, die nach dem vollständigen Einschieben der Halteplatte (6) in die Kopfplatte (4) auf deren Stirnflächen (42) aufliegen.

7. Operationshalterung nach einem der vorhergehenden Ansprüche, **dadurch gekennzeichnet, dass** die Halteplatte (6) wenigstens einen Stift aufweist, der in eine dazu komplementäre Bohrung in der Kopfplatte (4) einsteckbar und/oder einrastbar ist.

8. Operationshalterung nach einem der vorhergehenden Ansprüche, **dadurch gekennzeichnet, dass** die Kopfplatte (4) eine kugelsegmentförmige Ausnehmung aufweist, welche auf einen Kugelkopf in einer wählbaren Winkelposition fixiert werden kann.

9. Operationshalterung nach Anspruch 8, **dadurch gekennzeichnet, dass** der Kugelkopf
- Rippen oder
- Wülste oder
- Noppen
aufweist, die in dazu komplementäre Vertiefungen in der Ausnehmung der Halteplatte (6) eingreifen.

## Claims

1. Surgical fixture device, comprising a C- or U-shaped retaining bracket (2),
- which is suitable for extending on a part of the outer circumference of a body part (1), and
- is spaced apart from the body part (1) and
- bears at least one single pressure element (3) on the end of its first leg (21) and
- bears at least two pressure elements (3) on the end of its second leg (22) and
- all pressure elements (3) are suitable for being pressed onto the surface of the body part (1) or partly into the surface,
- the pressure elements (3) are fastened to at least one of the two legs (21, 22) by means of a head plate (4) in each case,
- and at least two threaded bores (5) having an internal thread in each case are provided, in which a pressure element (3) is fastened,
- into the head plate (4), a hollow profile (41) is formed, which is oriented approximately transversely to the pressure elements (3) and is shaped such that various types of retaining plate (6) can be slid in or plugged in,
**characterised in that** a retaining plate (6), into which at least two threaded bores (5) are introduced, is introduced into the hollow profile.

2. Surgical fixture device according to claim (1), **characterised in that** the body part is
- the head or
- an arm or
- a leg or
- the pelvis.

3. Surgical fixture device according to one of the preceding claims, **characterised in that** the pressure element (3) is
- a skull pin or
- a pressure plate or
- a pressure pad, such as a gel pad.

4. Surgical fixture device according to one of the preceding claims, **characterised in that** the hollow profile (41) is approximately U-shaped and its side walls have a depression in each case, which, in the longitudinal direction of the pressure elements (3), form an undercut.

5. Surgical fixture device according to claim (4), **characterised in that** the retaining plate (6), has, in cross-section, a profile that is at least partly complementary to the hollow profile (41), with bulges which are at least partly continuous and engage in the depressions of the side walls of the hollow profile (41).

6. Surgical fixture device according to claim (5), **characterised in that** the retaining plate (6) has at its two end faces resilient detent lugs (61), which, after complete sliding in of the retaining plate (6) into the head plate (4), lie against the end faces (42) thereof.

7. Surgical fixture device according to one of the preceding claims, **characterised in that** the retaining plate (6) has at least one pin, which can be plugged into and/or engaged in a bore in the head plate (4) that is complementary thereto.

8. Surgical fixture device according to one of the preceding claims, **characterised in that** the retaining plate (6) has a hemispherical recess, which can be fixed on a ball head in an optional angular position.

9. Surgical fixture device according to claim (8), **characterised in that** the ball head comprises
- ribs or
- beads or
- nubs,
which engage in complementary depressions in the recess of the retaining plate (6).

## Revendications

1. Support d'opération, consistant en un étrier de fixation (2) ayant à peu près la forme d'un C ou d'un U,
- qui est capable de suivre le tracé d'une partie de la circonférence extérieure d'un membre (1), qui
- se trouve à une certaine distance de ce membre (1), qui
- porte, à l'extrémité de sa première branche (21), au moins un seul élément de pression (3) et qui
- porte, à l'extrémité de sa seconde branche (22), au moins deux éléments de pression (3),
- tous les éléments de pression (3) pouvant être pressés sur la surface du membre (1) ou bien être en partie pressés dans la surface,
- les éléments de pression (3) étant fixés sur au moins une des deux branches (21), (22), chacune au moyen d'une plaque de tête (4),
- et au moins deux orifices filetés (5) possédant chacun un filetage intérieur dans lequel est fixé un élément de pression (3)
**caractérisé par le fait**
- un profil creux (41) orienté à peu près perpendiculairement par rapport aux éléments de pression (3) étant formé dans la plaque de tête (4), lequel profil est conçu de manière à ce que différents types de plaques de retenue (6) puissent être insérés ou enfichés, **caractérisé par le fait qu**'une plaque de retenue (6), dans laquelle sont pratiqués au moins deux orifices filetés (5), est placée dans le profil creux.

2. Support d'opération selon la revendication 1, **caractérisé par le fait que** le membre est
- la tête ou
- un bras ou
- une jambe ou
- le bassin.

3. Support d'opération selon une des revendications précédentes, **caractérisé par le fait que** l'élément de pression (3) est
- une broche de crâne ou bien
- une plaque de pression ou bien
- un coussin de pression, comme par exemple un coussin de gel.

4. Support d'opération selon une des revendications précédentes, **caractérisé par le fait que** le profil creux (41) possède à peu près la forme d'un U et que chacune de ses parois latérales présente un renfoncement qui forme une contre-dépouille dans le sens longitudinal des éléments de pression (3).

5. Support d'opération selon la revendication 4, **caractérisé par le fait que** la plaque de retenue (6) présente dans sa section un profil au moins en partie complémentaire au profil creux (41), avec au moins en partie des bombements traversants qui s'encrantent dans les renfoncements des parois latérales du profil en creux (41).

6. Support d'opération selon la revendication 5, **caractérisé par le fait que** la plaque de retenue (6) présente des nez d'encrantement amortisseurs sur ses deux faces frontales, qui reposent sur les surfaces frontales (42) une fois la plaque de retenue (6) entièrement insérée dans la plaque de tête (4).

7. Support d'opération selon une des revendications précédentes, **caractérisé par le fait que** la plaque de retenue (6) présente au moins une goupille, qui peut être enfichée et/ou encrantée dans un orifice complémentaire à cette dernière, pratiquée dans la plaque de tête (4).

8. Support d'opération selon une des revendications précédentes, **caractérisé par le fait que** la plaque de tête (4) présente une réservation en forme de segment de sphère qui peut être fixée dans une position d'angle pouvant être choisie.

9. Support d'opération selon la revendication 8, **caractérisé par le fait que** la tête sphérique présente
- des nervures ou bien
- un bourrelet ou bien
- des picots
qui s'encrantent dans les renfoncements complémentaires à cela dans la réservation de la plaque de retenue (6).
